# NEW EUROPEAN PATENT SPECIFICATION

(11) **EP 2 945 799 B2**
(45) Date of publication and mention of the opposition decision: **14.01.2026**
(45) Mention of the grant of the patent: 22.04.2020
(21) Application number: 14700755.3
(22) Date of filing: 17.01.2014
(51) Int. Cl.: B32B 27/08, B32B 27/30, B32B 27/32, B65D 65/40

(54) **MULTILAYER FILM COMPRISING A CONTACT LAYER, CORE LAYER AND OUTER LAYER FOR THE WALL OF A SINGLE-USE POUCH**
MEHRSCHICHTFOLIE MIT EINER KONTAKTSCHICHT, KERNSCHICHT UND AUSSENSCHICHT FÜR DIE WAND EINES EINWEGBEUTELS
FILM MULTICOUCHE COMPRENANT UNE COUCHE DE CONTACT, UNE COUCHE CENTRALE ET UNE AUTRE EXTÉRIEURE POUR LA PAROI D'UN SACHET À USAGE UNIQUE

(30) Priority: 18.01.2013 EP 13305068
(43) Date of publication of application: 25.11.2015
(73) Proprietor: SARTORIUS STEDIM FMT SAS, 13400 Aubagne (FR)
(72) Inventor: BARBAROUX, Magali, F-13112 La Destrousse (FR); DELAUNAY, Lucie, F-13360 Roquevaire (FR); HUSEMANN, Ute, 37079 Göttingen (DE); GRELLER, Gerhard, 37085 Göttingen (DE)
(74) Representative: Plasseraud IP
(86) International application number: PCT/EP2014/050954
(87) International publication number: WO 2014/111548

(56) References cited:
- EP-A1- 0 561 428
- WO-A1-2011/157813
- WO-A1-95/26268
- WO-A2-99/15289
- GB-A- 2 131 739
- US-A1- 2007 054 142
- US-A1- 2007 269 623
- US-A1- 2007 269 623
- US-A1- 2009 061 061
- US-A1- 2011 026 360
- US-A1- 2012 208 039
- SIMONE VIGAN�: "The Use of Metallocene Polyethylene in Co-Extruded Lamination Film", 14 May 2007 (2007-05-14), XP055506948, Retrieved from the Internet <URL:http://www.tappi.org/content/events/07europlace/papers/07europl48.pdf> [retrieved on 20180913]
- HAMMOND MATTHEW; NUNN HEATHER; ROGERS GARY; LEE HANS; MARGHITOIU ANATOLIA-LILIANA; PEREZ LOURDES; NASHED-SAMUEL YASSER; ANDERSON C: "Identification of a leachable compound detrimental to cell growth in single-use bioprocess containers.", PDA; JOURNAL OF PHARMACEUTICAL SCIENCE AND TECHNOLOGY, PARENTERAL DRUG ASSOCIATION, US, vol. 67, no. 2, 1 March 2013 (2013-03-01), US , pages 123 - 134, XP009185758, ISSN: 1948-2124, DOI: 10.5731/pdajpst.2013.00905
- SHUKLA ABHINAV A.; GOTTSCHALK UWE: "Single-use disposable technologies for biopharmaceutical manufacturing", TRENDS IN BIOTECHNOLOGY., ELSEVIER PUBLICATIONS, CAMBRIDGE., GB, vol. 31, no. 3, 21 November 2012 (2012-11-21), GB , pages 147 - 154, XP028985934, ISSN: 0167-7799, DOI: 10.1016/j.tibtech.2012.10.004
- JENKE, D.R. JENE, J.M. POSS, M. STORY, J. TSILIPETROS, T. ODUFU, A. TERBUSH, W.: "Accumulation of extractables in buffer solutions from a polyolefin plastic container", INTERNATIONAL JOURNAL OF PHARMACEUTICS, ELSEVIER, NL, vol. 297, no. 1-2, 13 June 2005 (2005-06-13), NL , pages 120 - 133, XP004903995, ISSN: 0378-5173

## Description

### Field of the invention

The invention relates to a multilayer film for the wall of a single-use pouch which may be used for preparing, storing or conveying a cell media or a cell culture, as well as a process for manufacturing such a multilayer film.

The invention also relates to the single-use pouch manufactured from that multilayer film and its process of manufacturing.

### Background

Single-use pouches are widely used in the field of cell culture, for instance in single-use bioreactors or for storing or conveying cell fluids, such as cell medium or cell culture.

Such pouches may comprise two large walls sealed to one another. Once expanded, they have a limited volume and remain relatively thin, which justifies the fact that they are often called 2D pouches (D meaning dimensions). 3D pouches are also known that comprise two end walls and a side wall that can be folded flat or deployed unfolded, sealed to one another, with the volume able to reach 3,000 liters, and even more. Such 3D pouches are described in the document WO00/04131 or are marketed by the company Sartorius under the trademark FLEXEL^{®} 3D.

The wall of a single-use pouch is generally composed of a multilayer film comprising a contact layer which in contact with the medium that fills the pouch, a barrier layer and an outer layer which is in contact with the external environment of the bag, the three layers being connected one to each other with a tie layer.

If the pouch is to be filled with a biopharmaceutical product, the contact layer should be made from a material that can be in contact with this biopharmaceutical product without causing degradation of the film and of the biopharmaceutical product. Furthermore, it must be sealable on itself. For that purpose, the material is generally selected from polyolefins, such as polyethylene.

The barrier layer provides a barrier to the passage of gases such as oxygen, carbon dioxide and is typically made from ethylene vinyl alcohol (EVOH).

The outer layer contributes to the mechanical strength of the pouch wall. For that purpose, it must be sufficiently flexible to withstand high mechanical stress but not be too much stretchable in order to prevent deformation of the pouch when it is filled with a product.

In the bioreactor field, the various systems mainly differ by their mode of stirring, which may involve a swinging movement, an orbital movement or an axial movement. Mechanical stresses may hence vary significantly from one system to another. In the liquid transport field, the mechanical stresses may also be very high. In both fields, a film which is not able to withstand these mechanical stresses may result in leakage of the product outside the pouch.

Therefore, there is a need for a film which is able to withstand various mechanical stresses in order to be usable in a wide range of applications, such as in bioreactors whatever the mode of stirring, for preparing a solution either by liquid/liquid or solid/liquid stirring, for storing or conveying a fluid in a 2D or 3D pouch, for both small or large volumes.

Moreover, in the biopharmaceutical field, the single-use pouches are to be sterilized before use. Sterilization is generally performed either by gamma irradiation or by ethylene oxide treatment. Therefore, the material used in the various films composing the pouch should be able to withstand gamma radiation or ethylene oxide treatment without degrading their physical properties.

In the case of pouches whose contact film is made from polyethylene, the applicant of the present patent application has found that when gamma irradiation was used to sterilize the pouch, the cellular growth of a cell culture prepared in that pouch could be slowed down, in particular due to a lag phase occurring at the beginning of the cell culture.

Following extensive testing, the applicant of the present application has further found that this lag phase was in part due to a compound which is released by the contact film into the culture medium, said compound resulting from the degradation of an anti-oxidant of said contact film during the gamma irradiation of the pouch.

Therefore, there is also a need for a film which has no or limited impact on the cellular growth of a culture medium further to sterilization by gamma irradiation. In particular, there is a need for a film which releases no significant amounts of degradation compound further to gamma irradiation, said degradation compound being able to interfere with cellular growth.

### Summary of the invention

The invention meets all these needs by providing a multilayer film for a single-use pouch, which shows remarkable mechanical properties, thereby allowing a wide range of applications, and which furthermore has no or limited impact on the cellular growth further to sterilization by gamma irradiation, thereby making it particularly suitable for preparing, storing or conveying a cellular medium. Furthermore, the multilayer film of the invention is made from components that can be combined easily according to the required structure.

One object of the present invention is a multilayer film for manufacturing a pouch, said multilayer film comprising:
- a contact layer,
- a core layer,
- an outer layer,
wherein:
- the contact layer and the core layer are made integral with a first tie layer,
- the outer layer and the core layer are made integral with a second tie layer,
- the contact layer comprises (A) a copolymer of ethylene and α-olefin having a density in the range of 0.870 g/cm³ to 0.910 g/cm³, alone or in mixture with (B) a polyolefin having a density in the range of 0.910 g/cm³ to 0.940 g/cm³,
- the core layer comprises (C') a flex-crack resistant ethylene vinyl alcohol copolymer having a content of ethylene in the range of 25 to 48 mol % or a mixture of (C) an ethylene vinyl alcohol copolymer having a content of ethylene in the range of 25 to 48 mol % and (D) an ionomer acid ethylene copolymer,
- the outer layer comprises (E) a polyolefin having a density in the range of 0.910 g/cm³ to 0.940 g/cm³, alone or in mixture with (F) a copolymer of ethylene and α-olefin having a density in the range of 0.870 g/cm³ to 0.910 g/cm³,
- the first tie layer and second tie layer comprise independently of each other (G) a copolymer of polyolefin grafted with a carboxylic acid or an anhydride of carboxylic acid, alone or in mixture with (H) a copolymer of polyolefin.

In one embodiment, copolymer (A) is a plastomer polyolefin having from 4 to 8 carbon atoms, in particular a linear low density copolymer of ethylene and oct-1-ene made from a process utilizing a metallocene catalyst.

In one embodiment, polyolefin (B) is a homopolymer of ethylene, in particular a low density polymer of ethylene.

In one embodiment, ethylene vinyl alcohol copolymer (C) is flex-crack resistant.

In one embodiment, copolymer (D) is an acrylic acid copolymer of ethylene ionomer wherein the carboxylate groups are associated with zinc cations.

In one embodiment, polyolefin (E) is a copolymer of ethylene with one or more olefin having from 4 to 8 carbon atoms, in particular a linear low density copolymer of ethylene and hexene.

In one embodiment, copolymer (F) is a plastomer polyolefin having from 4 to 8 carbon atoms, in particular a linear low density copolymer of ethylene and oct-1-ene made from a process utilizing a metallocene catalyst.

In one embodiment, copolymer (G) is a copolymer of ethylene grafted with maleic anhydride.

In one embodiment, copolymer (H) is a plastomer polyolefin having from 4 to 8 carbon atoms, in particular a linear low density copolymer of ethylene and oct-1-ene made from a process utilizing a metallocene catalyst.

In one embodiment, the contact layer comprises a mixture of (A) and (B) with a mass ratio (B)/(A) less than or equal to 95/5, preferably in the range of 5/95 to 95/5, more preferably in the range of 0.2 to 5, even more preferably in the range of 0.2 to 2.

In one embodiment, the core layer comprises a mixture of (C) an ethylene vinyl alcohol copolymer having a content of ethylene in the range of 25 to 48 mol % and (D) an ionomer acid ethylene copolymer with a mass ratio (C)/(D) in the range of 95/5 to 55/45, preferably in the range of 10 to 19, more preferably in the range of 15 to 19.

In one embodiment, the outer layer comprises a mixture of (E) and (F) with a mass ratio (E)/(F) higher than or equal to 5/95, preferably in the range of 5/95 to 100/0, more preferably in the range of 1 to 5, even more preferably in the range of 1 to 2.5.

In one embodiment, the first tie layer and second tie layer comprise independently of each other a mixture of (G) and (H) with a mass ratio (H)/(G) less than or equal to 95/5, preferably in the range of 5/95 to 95/5, more preferably in the range of range of 0.2 to 5, even more preferably in the range of 0.2 to 2.5.

In one embodiment, the contact layer contains no additives or limited amounts, preferably less than 0.10 wt %, more preferably less than 0.07 wt %, of additives able to release a degradation compound further to gamma irradiation that can slow down or delay cellular growth, such as Tris(2,4-ditert-butylphenyl) phosphite.

In one embodiment, the multilayer film of the invention further comprises one or several intermediate layers between two of any layers of the film.

Another object of the present invention is a process for manufacturing the multilayer film of the invention, comprising cast coextrusion of the contact layer, first tie layer, core layer, second tie layer and outer layer.

Another object of the present invention is a single-use pouch whose wall comprises the multilayer film of the invention.

Another object of the present invention is a bioreactor comprising the single-use pouch of the invention.

### Detailed description of the invention

Unless otherwise mentioned, the density of polymers referred to in the present application is as measured according to the Standard Test Method ASTM D792 - 08.

According to the present invention, the expression "made integral with" means that the two layers are attached together either directly or indirectly, i.e. by means of one or several intermediate layers. Therefore, the multilayer film of the invention may further comprise one or several intermediate layers between two of any layers of the film as defined above. Such intermediate layers may comprise a polymer chosen among one of the following polymers, or a mixture thereof:
- semi-crystalline thermoplastic polyolefins, such as polyethylene (PE), polypropylene (PP), polymethylpentene (PMP), or polybutene-1 (PB-1);
- elastomer polyolefins, such as polyisobutylene (PIB), ethylene-propylene (EPR or EPM), or ethylene-propylene-diene monomere (EPDM) ;
- Polyisobutylene (PIB) ;
- Polymers comprising ethylene as a comonomer, such as:
   - ethylene- vinyl acetate (EVA);
   - ethylene copolymers-acrylic esters, such as ethylene-methyl acrylate (EMA), ethylene-ethyl acrylate (EEA) ;
   - ethylene-acrylic ester-maleic anhydride (EEAMA); or
   - ethylene-polyvinylic alcohol (EVOH).

According to the invention, the expression "a layer comprises X" means that said layer comprises X in any amounts or is substantially composed of X.

### CONTACT LAYER

According to the present invention, the contact layer comprises (A) a copolymer of ethylene and α-olefin having a density in the range of 0.870 g/cm³ to 0.910 g/cm³, alone or in mixture with (B) a polyolefin having a density in the range of 0.910 g/cm³ to 0.940 g/cm³.

Preferably, the copolymer (A) has a density in the range of 0.890 g/cm³ to 0.905 g/cm³, preferably a density in the range of 0.895 g/cm³ to 0.905 g/cm³.

Preferably, the polyolefin (B) has a density in the range of 0.915 g/cm³ to 0.935 g/cm³, preferably a density in the range of 0.920 g/cm³ to 0.930 g/cm³.

As previously mentioned, the contact layer should be made from a material that can be in contact with a biopharmaceutical product without causing degradation of the film and of the biopharmaceutical product.

Furthermore, it must be sealable on itself. For that purpose, a copolymer of ethylene and α-olefin (A) having a density in the range of 0.870 g/cm³ to 0.910 g/cm³ may be chosen among polyolefin plastomers. These resins are made through either the single site or Ziegler-natta catalysis process. They may be chosen in particular among copolymers of ethylene with one or more α-olefins having at least 3 carbon atoms, preferably from 4 carbon atoms to 8 carbon atoms, such as but-1-ene, hex-1-ene or oct-1-ene, more particularly among copolymers of ethylene and octene, ethylene and hexene, ethylene and butene or ethylene and propylene. Copolymer of ethylene and α-olefin (A) is more preferably a linear low density copolymer of ethylene and oct-1-ene made from a process utilizing a metallocene catalyst, commonly referred as mLLDPE-C8.

Suitable copolymers (A) are sold by The Dow Chemical Company under the trademark AFFINITY^{®} or ENGAGE^{®}, or by ExxonMobil Chemical under the trademark EXACT^{®}.

In order to facilitate the processing of the contact layer and decrease its sticking effect, copolymer (A) may be mixed with a polyolefin (B) having a density in the range of 0.910 g/cm³ to 0.940 g/cm³. Polymer (B) may be chosen among homopolymers or copolymers of olefins, preferably among homopolymers of ethylene. Polyolefin (B) is more preferably a low density polymer of ethylene, commonly referred as LDPE. Suitable polyolefins (B) are sold for instance by LyondellBasel Industries under the trademark LUPOLEN^{®} (more precisely LUPOLEN^{®} 2426HK) or The DOW Chemical Company under the name DOW LDPE^{®}.

The flexibility, sealability and sticking effect of the contact layer can be controlled by adjusting the proportion between (A) and (B).

In one embodiment, the contact layer comprises a mixture of (A) and (B) with a mass ratio (B)/(A) less than or equal to 95/5, preferably in the range of 5/95 to 95/5.

For a good compromise between flexibility, sealability and sticking effect, and thus cover a wide range of applications, the mass ratio (B)/(A) may be in the range of 0.2 to 5, preferably in the range of 0.2 to 2.

The contact layer may contain one or more conventional additives to protect the polymers such as antioxydants (- Octadecyl 3-(3,5-di-tert-butyl-4-hydroxyphenyl)propionate - Pentaerythritol tetrakis(3-(3,5-di-tert-butyl-4-hydroxyphenyl)propionate),- Tris(2,4-ditert-butylphenyl) phosphite,- Butylhydroxytoluene,-1,3,5,Tris(3,5-di-tert-butyl-4-hydrox benzyl)-s-triazine-2,4,6-(1H,3H,5H)trione, - Ethylene bis[3,3-bis[3-(1,1-dimethylethyl)-4-hydroxyphenyl]butanoate]) or to help processing of the layer, such as anti-blocks (such as silicon dioxide, magnesium silicate, calcium carbonate, calcium stearate, ethylene bisstearamide, stearyl erucamide, stearamide, erucamide, glycerol monostearate, zinc stearate, or silicone). Preferably the additives should be compliance with the European pharmacopoeia 6.0, 3.1.3 Polyolefines or 3.1.5 Polyethylene with additives for containers for parenteral preparations and for ophthalmic preparations. Preferably, the amount of the additives in the contact layer should be less than 0.50 wt %, preferably less than 0.10 wt%, more preferably less than 0.07 wt%.

If the contact layer is intended to be in contact with a cell medium, the contact layer should not contain slip agents or phthalate as plasticizers because these additives may contain small molecular weight molecules able to move to the surface of the layer, thereby contaminating the cell medium.

Furthermore, the contact layer should contain no additives or limited amounts (less than 0.10 wt %, preferably less than 0.07 wt%) of additives able to release a degradation compound further to gamma irradiation in the range of 25 kGy - 50 kGy that can slow down or delay cellular growth.

Such degradation compounds able to slow down or delay cellular growth may be detected by testing the film in a Biostat^{®} Cultibag RM system from Sartorius Stedim Biotech during 7 days, with Per-C6 cells line, by comparing with a control film (which does not delay or slow down cellular growth), such as EVA film BF1400 marketed by Renolit.

It is considered that cellular growth is slowed down or delayed when the lag phase is at least 24 hours.

Such degradations compounds may be released from antioxidants. Antioxidants are used to prevent polymer degradation which may be initiated by heat, light, impurities such as catalyst residue, or mechanical stress.

The contact layer preferably contains no antioxidant or limited amounts of antioxidant, less than 0.3 wt % (preferably less than 0.10 wt %, more preferably less than 0.07 wt%). Table 1 provides examples of such oxidants.

**Table 1**

| Chemical name | CAS n° | Chemical formula |
|---|---|---|
| Octadecyl 3-(3,5-di-tert-butyl-4-hydroxyphenyl)propionate | CAS n° 2082-79-3 | 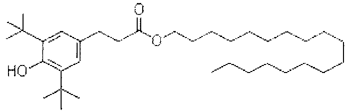 |
| Pentaerythritol tetrakis(3-(3,5-di-tert-butyl-4-hydroxyphenyl)propionate) | CAS n° 6683-19-8 | 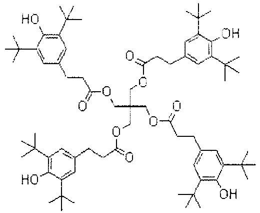 |
| Tris(2,4-ditert-butylphenyl) phosphite | CAS n° 31570-04-4 | 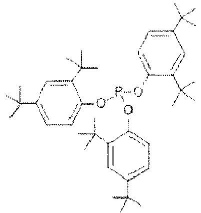 |
| Butylhydroxytoluène | CAS n° 128-37-0 | 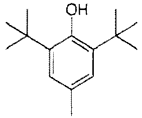 |
| 1,3,5,Tris(3,5-di-tert-butyl-4-hydroxybenzyl)-s-triazine-2,4,6-(1 H, 3H,5H)trione | CAS n° 27676-62-6 | 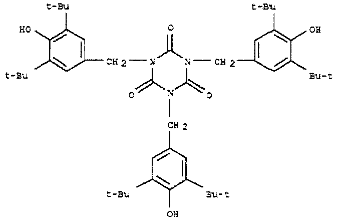 |
| Ethylene bis[3,3-bis[3-(1,1-dimethylethyl)-4-hydroxyphenyl] butanoate] | CAS n° 32509-66-3 | 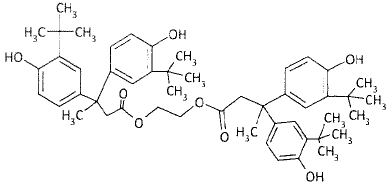 |

The antioxidants are classified into two types, primary and secondary, depending on the mechanism used to halt the degradation process. Degradation compounds able to slow down or delay cellular growth can be released from primary and secondary antioxidants, such as phenolic antioxidants or phosphite antioxidant. In particular, the contact layer contains no or limited amounts (less than 0.01 wt %) of a secondary antioxidant, preferably of a phosphite antioxidant, such as tris(2,4-ditert-butylphenyl) phosphite.

Under gamma irradiation, tris(2,4-ditert-butylphenyl) phosphite is converted into a phosphate, which in turns releases degradation compounds as follows:

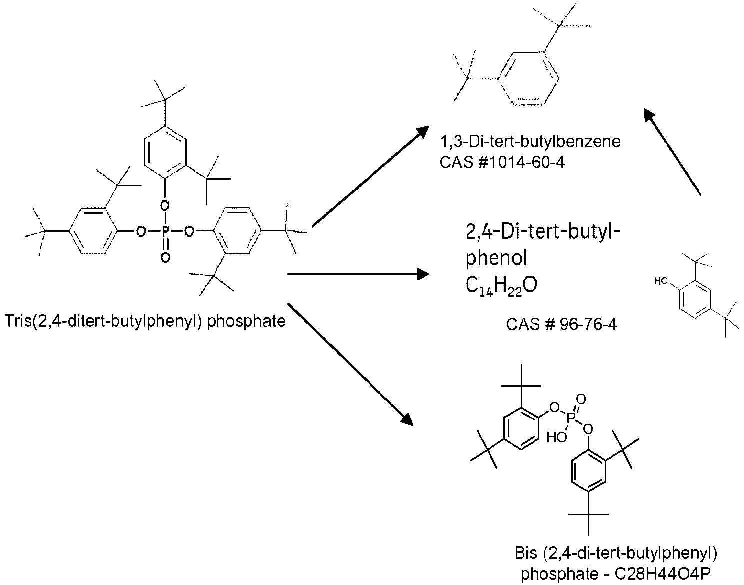

The thickness of the contact layer is from 150 µm and 300 µm, preferably from 200 and 250 µm, more preferably from 225 µm and 245 µm.

The contact layer may be manufactured by extrusion. Preferably, the contact layer is manufactured by cast extrusion with the other layers of the multilayer film, without the use of silicone in order to make the contact layer inert with regard to a cell medium.

### CORE LAYER

According to the present invention, the core layer comprises (C') a flex-crack resistant ethylene vinyl alcohol copolymer having a content of ethylene in the range of 25 to 48 mol % or a mixture of (C) an ethylene vinyl alcohol copolymer having a content of ethylene in the range of 25 to 48 mol % and (D) an ionomer acid ethylene copolymer.

The flex crack resistance is measured according to the Standard Test Method for Flex Durability of Flexible Barrier Materials ASTM F392 (2004). According to the present invention, a material is flex-crack resistant if the number of pine holes measured after 100 cycles according to the method of ASTM F392 (film sample : 30 µm mono layer, test condition: 23°C, Gelbo flex tester: 440° twisting motion) is less than or equal to 30. It was found that the introduction of such a polymer into the core layer makes the multilayer film more resistant to mechanical stresses.

The ethylene vinyl alcohol copolymer (C) or (C') having a content of ethylene in the range of 25 to 48 mol % is used to provide a barrier to the passage of gases such as oxygen, carbon dioxide. Ethylene vinyl alcohol copolymers are commonly abbreviated EVOH. Preferably, the content of ethylene in copolymer (C) or (C') is in the range of 29 to 44 mol %, most preferably around 29 to 38 mol %. Suitable EVOH whose ethylene content is from 25 to 48 mol %are sold for instance by Nippon Goshei under the trademark SOARNOL^{®}. Suitable EVOH whose ethylene content is from 27 to 48 mol % are sold for instance by Kuraray under the trademark EVAL^{®}.

Advantageously, the copolymer (C) is flex-crack resistant.

Preferably, the flex crack resistant polymer (C) or (C') is such that its barrier properties are maintained even during or after torsion or twisting application movements.

The lower the content of ethylene in copolymer (C) or (C') is, the higher the gas barrier effect of the layer is, but at the same time the more rigid the core layer is. If the core layer is too rigid, it may crack or cause delamination within the multilayer film.

In order to limit this risk of cracking or delamination, copolymer (C) is mixed with an ionomer acid ethylene copolymer (D) which helps to absorb the mechanical stresses of the barrier layer. The copolymer (D) is particularly suitable because it mixes readily with copolymer (C) and displays good affinity with the tie layers.

By "ionomer acid ethylene copolymer", it is meant acid ethylene copolymer partially neutralized with metal salts such as zinc or sodium ions. Suitable copolymers (D) include acrylic acid copolymers of ethylene ionomers, wherein the carboxylate groups are associated with zinc cations. Such copolymers are sold for instance by DuPont Packaging & Industrial Polymers under the trademark SURLYN^{®} or by The Dow Chemical Company under the trademark AMPLIFY^{®} IO.

The flexibility and gas barrier effect of the film can be controlled by adjusting the proportion between (C) and (D).

In one embodiment, the core layer comprises a mixture of (C) an ethylene vinyl alcohol copolymer having a content of ethylene in the range of 25 to 48 mol % and (D) an ionomer acid ethylene copolymer with a mass ratio (C)/(D) in the range of 95/5 to 55/45.

For a good compromise between flexibility and gas barrier effect, and thus cover a wide range of applications, the mass ratio (C)/(D) may be in the range of 10 to 19, preferably in the range of 15 to 19.

The thickness of the core layer is from 5 µm and 50 µm, preferably from 15 and 40 µm, more preferably from 20 µm and 30 µm.

The core layer may be manufactured by extrusion. Preferably, the core layer is manufactured by cast extrusion with the other layers of the multilayer film, without the use of silicone in order to make the contact layer inert with regard to a cell medium.

### OUTER LAYER

According to the invention, the outer layer comprises (E) a polyolefin having a density in the range of 0.910 g/cm³ to 0.940 g/cm³, alone or in mixture with (F) a copolymer of ethylene and α-olefin having a density in the range of 0.870 g/cm³ to 0.910 g/cm³.

Preferably, the polyolefin (E) has a density in the range of 0.925 g/cm³ to 0.940 g/cm³, more preferably a density in the range of 0.930 g/cm³ to 0.940 g/cm³.

Preferably, the copolymer (F) has a density in the range of 0.890 g/cm³ to 0.905 g/cm³, more preferably a density in the range of 0.895 g/cm³ to 0.905 g/cm³.

Polyolefins (E) having a density in the range of 0.910 g/cm³ to 0.940 g/cm³ may be chosen among homopolymers or copolymers of olefins, preferably among copolymers of ethylene. A linear low density copolymer of ethylene is particularly suitable because it shows a better mechanical resistance to tensile stress. Preferred polyolefins (E) include copolymers of ethylene with one or more olefins having at least 3 carbon atoms, preferably from 4 carbon atoms to 8 carbon atoms, such as butene, hexene or octene. Polyolefin (E) is more preferably a linear low density copolymer of ethylene and hexene, commonly referred as LLDPE-C6. Suitable polyolefins (E) are sold for instance by INEOS under the name INEOS LLDPE (more precisely INEOS LLDPE LL6930AA) or by the DOW Chemical Company under the trademark DOWLEX^{™} (more precisely DOWLEX^{™} SC 2108G).

Copolymers of ethylene and α-olefin (F) having a density in the range of 0.870 g/cm³ to 0.910 g/cm³ may be chosen among polyolefin plastomers. These resins are made through either the single site or Ziegler-natta catalysis process. They may be chosen in particular among copolymers of ethylene with one or more α-olefins having at least 3 carbon atoms, preferably from 4 carbon atoms to 8 carbon atoms, such as but-1-ene, hex-1-ene or oct-1-ene, more particularly among copolymers of ethylene and octene, ethylene and hexene, ethylene and butene or ethylene and propylene. Copolymer of ethylene and α-olefin (F) is more preferably a linear low density copolymer of ethylene and oct-1-ene made from a process utilizing a metallocene catalyst, commonly referred as mLLDPE-C8. Suitable polyolefins (F) are sold for instance by The Dow Chemical Company under the trademark AFFINITY^{®} or ENGAGE^{®}, or by ExxonMobil Chemical under the trademark EXACT^{®}.

The outer layer contributes to the mechanical strength of the pouch wall. For that purpose, it must be sufficiently flexible to withstand high mechanical stress but not be too much stretchable, i.e. sufficiently rigid, in order to prevent deformation of the pouch when it is filled with a product.

The polyolefin (E) gives to the film the desired rigidity, while copolymer (F) gives to the film the resistance to mechanical strength, in particular resistance to twist stress. The flexibility and rigidity of the film can be controlled by adjusting the right proportion between (E) and (F).

In some applications where the film should be able to resist to the pressure of the liquid contained in the pouch (for instance with high volumes of liquid), it is preferable to have an amount of polyolefin (E) which is higher than copolymer (F).

In other applications, where the film should withstand flexion and mechanical stress, it may be preferable to have an amount of copolymer (F) which is higher than polyolefin (E).

In one embodiment, the outer layer comprises a mixture of (E) and (F) with a mass ratio (E)/(F) higher than or equal to 5/95, preferably in the range of 5/95 to 100/0.

For a good compromise between flexibility and rigidity, and thus cover a wide range of applications, the mass ratio (E)/(F) may be in the range of 1 to 5, preferably in the range of 1 to 2.5.

The outer layer may contain one or more conventional additives to protect the polymers such as antioxydants (- Octadecyl 3-(3,5-di-tert-butyl-4-hydroxyphenyl)propionate - Pentaerythritol tetrakis(3-(3,5-di-tert-butyl-4-hydroxyphenyl)propionate),- Tris(2,4-ditert-butylphenyl) phosphite,- Butylhydroxytoluène,-1,3,5,Tris(3,5-di-tert-butyl-4-hydrox benzyl)-s-triazine-2,4,6-(1H,3H,5H)trione, - Ethylene bis[3,3-bis[3-(1,1-dimethylethyl)-4-hydroxyphenyl]butanoate]) or to help processing of the layer, such as anti-blocks (such as silicon dioxide, magnesium silicate, calcium carbonate, calcium stearate, ethylene bisstearamide, stearyl erucamide, stearamide, erucamide, glycerol monostearate, zinc stearate, or silicone). Preferably the additives should be compliance with the European pharmacopoeia 6.0, 3.1.3 Polyolefines or 3.1.5 Polyethylene with additives for containers for parenteral preparations and for ophthalmic preparations. Preferably, the amount of the additives in the outer layer should be less than 0.50 wt %, preferably less than 0.10 wt%, more preferably less than 0.07 wt%.

Preferably, the additives are chosen among compositions which do not contain any compound susceptible to degradation under gamma irradiation.

The thickness of the outer layer is from 50 µm and 150 µm, in particular from 55 µm to 150 µm, preferably from 80 and 120 µm, more preferably from 90 µm and 110 µm.

The outer layer may be manufactured by extrusion. Preferably, the outer layer is manufactured by cast extrusion with the other layers of the multilayer film, without the use of silicone in order to make the contact layer inert with regard to a cell medium.

### TIE LAYER

According to the present invention, the first tie layer and second tie layer comprise independently of each other (G) a copolymer of polyolefin grafted with a carboxylic acid or an anhydride of carboxylic acid, alone or in mixture with (H) a copolymer of polyolefin.

The tie layer is able to seal the contact layer, core layer and outer layer between each other and to prevent separation of these layers during the use of the pouch.

In one embodiment, polyolefin (G) has a density in the range of 0.875 g/cm³ to 0.940 g/cm³.

In one embodiment, polyolefin (H) has a density in the range of 0.870 g/cm³ to 0.910 g/cm³.

In one embodiment, the first tie layer and second tie layer have identical compositions.

The binding properties are conferred by the copolymer (G) which is a copolymer of polyolefin grafted with a carboxylic acid or an anhydride of carboxylic acid. The copolymer of polyolefin grafted with a carboxylic acid or an anhydride of carboxylic acid is preferably a copolymer of ethylene, more particularly a copolymer of ethylene with one or more olefins having at least 3 carbon atoms, preferably from 4 carbon atoms to 8 carbon atoms, such as butene, hexene or octene, grafted with maleic acid anhydride. More preferably, the polymer (G) is a copolymer of ethylene grafted with maleic anhydride. Suitable polymers (G) are sold for instance by Dupont under the trademark BYNEL^{®}, or by The Dow Chemical Company under the trademark AMPLIFY^{™}TY or AMPLIFY^{™}GR.

For a better affinity with the contact layer, core layer and outer layer, and for a better flexibility of the film, the polymer (G) is mixed with copolymer (H).

Copolymer (H) may be chosen among polyolefin plastomers. These resins are made through either the single site or Ziegler-natta catalysis process. They may be chosen in particular among copolymers of ethylene with one or more α-olefins having at least 3 carbon atoms, preferably from 4 carbon atoms to 8 carbon atoms, such as but-1-ene, hex-1-ene or oct-1-ene, more particularly among copolymers of ethylene and octene, ethylene and hexene, ethylene and butene or ethylene and propylene. Copolymer of ethylene and α-olefin (H) is more preferably a linear low density copolymer of ethylene and oct-1-ene made from a process utilizing a metallocene catalyst, commonly referred as mLLDPE-C8. Suitable polyolefins (H) are sold for instance by The Dow Chemical Company under the trademark AFFINITY^{®} or ENGAGE^{®}, or by ExxonMobil Chemical under the trademark EXACT^{®}.

In one embodiment, the first tie layer and second tie layer comprise independently of each other a mixture of (G) and (H) with a mass ratio (H)/(G) less than 95/5, preferably in the range of 5/95 to 95/5.

For a good compromise between flexibility and sealability, and thus cover a wide range of applications, the mass ratio (H)/(G) may be in the range of range of 0.2 to 5, preferably in the range of 0.2 to 2.5.

The thickness of the tie layer is from 5 µm and 50 µm, preferably from 10 and 30 µm, more preferably from 15 µm and 25 µm.

The tie layer may be manufactured by extrusion. Preferably, the tie layer is manufactured by cast extrusion with the other layers of the multilayer film, without the use of silicone in order to make the contact layer inert with regard to a cell medium.

### MULTILAYER FILM

The multilayer film of the invention comprises a contact layer, a core layer and an outer layer, wherein the contact layer and the core layer are made integral with a first tie layer and the outer layer and the core layer are made integral with a second tie layer, and wherein the layers are as defined previously.

In one particular embodiment, the multilayer film of the invention comprises:
- a contact layer,
- a core layer,
- an outer layer,
- two tie layers
wherein:
- the contact layer and the core layer are made integral with a first tie layer,
- the outer layer and the core layer are made integral with a second tie layer,
- the contact layer comprises (A) a polyolefin plastomer having from 4 to 8 carbon atoms, such as linear low density copolymer of ethylene and oct-1-ene made from a process utilizing a metallocene catalyst, said polyolefin plastomer having a density in the range of 0.895 g/cm³ to 0.905 g/cm³,
- the core layer comprises:
   ∘ a mixture of (C) an ethylene vinyl alcohol copolymer having a content of ethylene in the range of 25 to 48 mol % and (D) an ionomer acid ethylene copolymer,
   ∘ with a mass ratio (C)/(D) in the range of 15 to 19,
- the outer layer comprises (E) a linear low density copolymer of ethylene and hexene having a density in the range of 0.930 g/cm³ to 0.940 g/cm³,
- the first tie layer and second tie layer comprise independently of each other (G) a copolymer of ethylene grafted with maleic anhydride.

In a preferred embodiment, copolymer (C) of the above defined multilayer film is flex-crack resistant.

In one particular embodiment, the multilayer film of the invention comprises:
- a contact layer,
- a core layer,
- an outer layer,
- two tie layers
wherein:
- the contact layer and the core layer are made integral with a first tie layer,
- the outer layer and the core layer are made integral with a second tie layer,
- the contact layer comprises (A) a polyolefin plastomer having from 4 to 8 carbon atoms, such as linear low density copolymer of ethylene and oct-1-ene made from a process utilizing a metallocene catalyst, said polyolefin plastomer having a density in the range of 0.895 g/cm³ to 0.905 g/cm³,
- the core layer comprises:
   o (C') a flex-crack resistant ethylene vinyl alcohol copolymer having a content of ethylene in the range of 25 to 48 mol %,
- the outer layer comprises (E) a linear low density copolymer of ethylene and hexene having a density in the range of 0.930 g/cm³ to 0.940 g/cm³,
- the first tie layer and second tie layer comprise independently of each other (G) a copolymer of ethylene grafted with maleic anhydride.

In another particular embodiment, the multilayer film of the invention comprises:
- a contact layer,
- a core layer,
- an outer layer,
- two tie layers
wherein:
- the contact layer and the core layer are made integral with a first tie layer,
- the outer layer and the core layer are made integral with a second tie layer,
- the contact layer comprises:
   ∘ a mixture of (A) a polyolefin plastomer having from 4 to 8 carbon atoms, in particular a linear low density copolymer of ethylene and oct-1-ene made from a process utilizing a metallocene catalyst, said polyolefin plastomer having a density in the range of 0.895 g/cm³ to 0.905 g/cm³ and (B) a low density polymer of ethylene having a density in the range of 0.920 g/cm³ to 0.930 g/cm³,
   ∘ with a mass ratio (B)/(A) in the range of 0.2 to 2,
- the core layer comprises:
   ∘ a mixture of (C) an ethylene vinyl alcohol copolymer having a content of ethylene in the range of 25 to 48 mol % and (D) an ionomer acid ethylene copolymer,
   ∘ with a mass ratio (C)/(D) in the range of 15 to 19,
- the outer layer comprises (E) a linear low density copolymer of ethylene and hexene having a density in the range of 0.930 g/cm³ to 0.940 g/cm³,
- the first tie layer and second tie layer comprise independently of each other:
   ∘ a mixture of (G) a copolymer of ethylene grafted with maleic anhydride and (H) a polyolefin plastomer having from 4 to 8 carbon atoms, in particular a linear low density copolymer of ethylene and oct-1-ene made from a process utilizing a metallocene catalyst,
   ∘ with a mass ratio (H)/(G) in the range of 0.2 to 2.5.

In a preferred embodiment, the copolymer (C) of the above defined multilayer film is flex-crack resistant.

In another particular embodiment, the multilayer film of the invention comprises:
- a contact layer,
- a core layer,
- an outer layer,
- two tie layers
wherein:
- the contact layer and the core layer are made integral with a first tie layer,
- the outer layer and the core layer are made integral with a second tie layer,
- the contact layer comprises:
   ∘ a mixture of (A) a polyolefin plastomer having from 4 to 8 carbon atoms, in particular a linear low density copolymer of ethylene and oct-1-ene made from a process utilizing a metallocene catalyst, said polyolefin plastomer having a density in the range of 0.895 g/cm³ to 0.905 g/cm³ and (B) a low density polymer of ethylene having a density in the range of 0.920 g/cm³ to 0.930 g/cm³,
   ∘ with a mass ratio (B)/(A) in the range of 0.2 to 2,
- the core layer comprises:
   ∘ a mixture of (C) an ethylene vinyl alcohol copolymer having a content of ethylene in the range of 25 to 48 mol % and (D) an ionomer acid ethylene copolymer,
   ∘ with a mass ratio (C)/(D) in the range of 15 to 19,
- the outer layer comprises:
   ∘ a mixture of (E) a linear low density copolymer of ethylene and hexene having a density in the range of 0.930 g/cm³ to 0.940 g/cm³ and (F) a polyolefin plastomer having from 4 to 8 carbon atoms, in particular a linear low density copolymer of ethylene and oct-1-ene made from a process utilizing a metallocene catalyst having a density in the range of 0.895 g/cm³ to 0.905 g/cm³,
   ∘ with a mass ratio (E)/(F) in the range of 1 to 2.5,
- the first tie layer and second tie layer comprise independently of each other:
   ∘ a mixture of (G) a copolymer of ethylene grafted with maleic anhydride and (H) a polyolefin plastomer having from 4 to 8 carbon atoms, in particular a linear low density copolymer of ethylene and oct-1-ene made from a process utilizing a metallocene catalyst,,
   ∘ with a mass ratio (H)/(G) in the range of 0.2 to 2.5.

In a preferred embodiment, the copolymer (C) of the above defined multilayer film is flex-crack resistant.

The layers may be processed into a multilayer film by standard extrusion techniques well known by the person skilled in the art including extrusion or coextrusion such as cast or blow extrusion, extrusion coating, extrusion coating and lamination or a combination thereof, for instance by co-extruding at least two layers and then coating on another layer, or by coextruding at least two layers, extruding another layer and then coating and laminating the coextruded layer and extruded layer together.

Preferably, the multilayer film is manufactured by using a cast coextrusion process.

Another object of the present invention is thus a process for manufacturing the multilayer film as described above, comprising cast coextrusion of the contact layer, first tie layer, core layer, second tie layer and outer layer.

The process should be conducted free of slip agents and other low molecular weight additives that may increase the extractables to an unacceptable level.

The multilayer film according to the invention is particularly suited for manufacturing single-use pouches, including 2D pouches or 3D pouches.

Another object of the present invention is thus a single-use pouch whose wall comprises the multilayer film as described above.

For that purpose, the thickness of the multilayer film is from 200 µm and 500 µm, preferably from 300 and 450 µm, more preferably from 350 µm and 450 µm.

The multilayer film of the invention is able to withstand various mechanical stresses making it usable in a wide range of applications, such as in bioreactors whatever the mode of stirring, for preparing a solution either by liquid/liquid or solid/liquid stirring, for storing or conveying a fluid in a 2D or 3D pouch, for both small or large volumes.

Such pouches may be manufactured according to standard techniques well known by the person skilled in the art.

The multilayer film of the invention is particularly suited for manufacturing single-use pouches intended to contain a cell medium or a cell culture as it has no or limited impact on the cellular growth of a culture medium further to sterilization by gamma irradiation. In particular, the contact layer of the multilayer film of the invention releases no or limited amount of degradation compound further to gamma irradiation, said degradation compound being able to interfere with cellular growth.

### Brief description of the figures

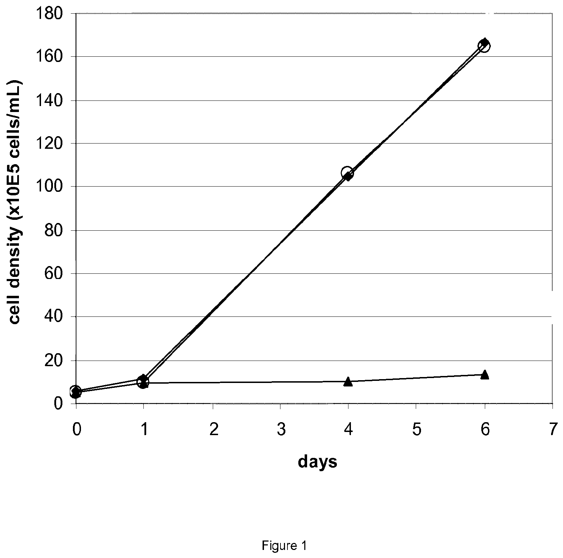

Figure 1 represents the cell density of a culture medium as a function of time, said culture medium being grown in a pouch made with a multilayer film according to the invention, compared with a culture medium grown in a pouch of the prior art (EVA contact layer).

The invention will now be further described in the following examples. These examples are offered to illustrate the invention and should in no way be viewed as limiting the invention.

### Example 1: Multilayer film

A multilayer film with a thickness of 400 µm was prepared and coextruded in accordance with the teaching of the present invention. The multilayer film has:
- an outer layer with a thickness of 100 µm comprising 98 wt% of LLDPE-C6 having a density of 0.936 g/cm³ and 2 wt% of an anti-block resin,
- a core layer with a thickness of 25 µm comprising 95 wt % of EVOH having an ethylene contain of 38%mol and 5 wt % of PE ionomer zinc having a density of 0.950 g/cm³,
- a contact layer with a thickness of 235 µm comprising 68 wt % of mLLDPE-C8 having a density of 0.902 g/cm³, 30 wt % of LDPE without additives having a density of 0.923 g/cm³ and 2 wt% of an anti-block resin,
- a first tie layer between the contact layer and the core layer, said tie layer comprising 70 wt % of mLLDPE-C8 having a density of 0.902 g/cm³ and 30 wt % LLDPE-MAH,
- a second tie layer between the contact layer and the core layer, said tie layer comprising 70 wt % of mLLDPE-C8 having a density of 0.902 g/cm³ and 30 wt % of LLDPE-MAH.

LLDPE-C6 = linear low density copolymer of ethylene and hexene.
EVOH = ethylene vinyl alcohol polymer.
PE ionomer zinc = acrylic acid copolymers of ethylene ionomer, wherein the carboxylate groups are associated with zinc cations.
mLLDPE-C8 = linear low density copolymer of ethylene and oct-1-ene made from a process utilizing a metallocene catalyst.
LLDPE-MAH = linear low density copolymer of ethylene grafted with maleic anhydride.
LDPE = low density polymer of ethylene.

### Example 2: Mechanical stress tests

### 2.1. Stirring table with orbital movement

A 2D pouch made with the multilayer film of example 1 (film No. 1), having a volume of 10 liters was filled with 10 liters of water and placed on a stirring table having an orbital movement. The stirring speed was 140 rpm. The test was performed until leakage of the fluid out of the pouch. Leakage occurred after 1.1 hours (standard deviation = 0.3).

The same 2D pouch made with the same multilayer film as the one of film No. 1, except that the core layer contained EVOH flex-crack resistant having a density of 1.12 g/cm³ (film No. 2), was tested under the same conditions. Leakage occurred after 1.5 hours (standard deviation = 0.3).

The same 2D pouch made with the same multilayer film as the one of film No. 2, except that the outer layer contained 30 wt % of mLLDPE-C8 having a density of 0.902 g/cm³ (film No. 3), 68 wt% of LLDPE-C6 having a density of 0.936 g/cm³ and 2 wt% of an anti-block resin, was tested under the same conditions. Leakage occurred after 2.1 hours (standard deviation = 0.7).

This example shows that the use of a flex-crack resistant EVOH into the core layer makes the film be more resistant to mechanical stress (time until leakage is increased by 36%).

This example further shows that the addition of a copolymer of ethylene and α-olefin of low density (in the range of 0.870 g/cm³ to 0.910 g/cm³, in particular of 0.902 g/cm³) to a polyolefin having a higher density into the outer layer makes the film be even more resistant to mechanical stress (time until leakage is increased by 40%).

The use of a flex-crack resistant EVOH into the core layer and the addition of a copolymer of ethylene and α-olefin of low density into the outer layer increases the time until which leakage occurs by 90%.

### 2.2. Rocking motion

Three pouches having a volume of 200 liters were made with film No. 1, film No. 2 and film No. 3 as described above, were filled with 100 liters of water and moved under a speed of 20 rpm and an angle of 10°. The test was performed during 21 days.

Regarding the pouch made with film No. 1, leakage occurred after 5 days.

Regarding the pouch made with film No. 2 and 3, no leakage occurred after 21 days.

This example corroborates the results obtained in example 2.1).

### Example 3: cell density

Three bags of 10 liters were manufactured with three different films A, B and C.

Film A is available on the market under the reference BF1400 (Renolit). It comprises an EVA contact layer.

Films B and C are multilayer films in accordance with the invention comprising a contact layer, a core layer, an outer layer, and 2 tie layers. Films B and C are identical except in the contact layer.

Film B has a thickness of 400 µm.Its contact layer has a thickness of 235 µm and comprises:
- mLLDPE-C8 having a density of 0.902 g/cm³ and
- less than 0,12 wt% of additives able to release a degradation compound further to gamma irradiation that can slow down or delay cellular growth.

Film C has a thickness of 400 µm. Its contact layer has a thickness of 235 µm and comprises:
- a mixture of mLLDPE-C8 having a density of 0.902 g/cm³ and LDPE without additives having a density of 0.923 g/cm³,
- a maximum of 0,06 wt% of additives able to release a degradation compound further to gamma irradiation that can slow down or delay cellular growth.

mLLDPE-C8 = linear low density copolymer of ethylene and oct-1-ene made from a process utilizing a metallocene catalyst.
LDPE = low density polymer of ethylene (INEOS ELTEX MED PH23H630 by INEOS).

The cell culture was performed in a Cultibag RM 10L Optical design made with the above described three films and gamma irradiated at 25-40kGy.

The cells culture was performed in parallel with Biostat^{®} Cultibag RM systems from Sartorius Stedim Biotech during 7 days, with Per-C6 cells line.

The cell density was measured by sampling at different dates in order to follow the growth behaviour.

Figure 1 represents the cell density as a function of time: 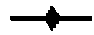 Cell culture in a bag made with a film A 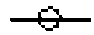 Cell culture in a bag made with a film C 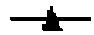 Cell culture in a bag made with a film B

### Results:

All along the cell culture, the viability of the cells was above 80%.

The cell culture performed in the bag made with the film A shows a good growth.

The cell culture performed in the bag made with the film B shows a bad growth.

The cell culture performed in the bag made with the film C shows good growth.

Figure 1 shows that the film B delays the cellular growth while film C has not impact on the cellular growth. From these results it is considered that the delay on cellular growth is due to the content of additives in the contact layer. These additives are able to release degradation compounds further to gamma irradiation that slow down or delay cellular growth.

Therefore, it is preferable to limit the amount of additives able to release such degradation compounds further to gamma irradiation in the contact layer.

The films of the invention has no impact on cellular growth both shortly after gamma irradiation (within one week) and several months after gamma irradiation, which may not be the case for film A.

## Claims

1. A multilayer film for manufacturing a pouch, said multilayer film comprising:
- a contact layer,
- a core layer,
- an outer layer,
wherein:
- the contact layer and the core layer are made integral with a first tie layer,
- the outer layer and the core layer are made integral with a second tie layer,
- the contact layer comprises (A) a copolymer of ethylene and α-olefin having a density in the range of 0.870 g/cm³ to 0.910 g/cm³, alone or in mixture with (B) a polyolefin having a density in the range of 0.910 g/cm³ to 0.940 g/cm³,
- the core layer comprises (C') a flex-crack resistant ethylene vinyl alcohol copolymer having a content of ethylene in the range of 25 to 48 mol % or a mixture of (C) an ethylene vinyl alcohol copolymer having a content of ethylene in the range of 25 to 48 mol % and (D) an ionomer acid ethylene copolymer, wherein the ethylene vinyl alcohol copolymer is flex-crack resistant if the number of pinholes measured after 100 cycles according to the method of ASTM F392 (2004) is less than or equal to 30, the test being performed on a film sample which is a 30 µm mono layer, at 23°C, with a Gelbo flex tester and a 440° twisting motion,
- the outer layer comprises (E) a polyolefin having a density in the range of 0.910 g/cm³ to 0.940 g/cm³, alone or in mixture with (F) a copolymer of ethylene and α-olefin having a density in the range of 0.870 g/cm³ to 0.910 g/cm³,
- the first tie layer and second tie layer comprise independently of each other (G) a copolymer of polyolefin grafted with a carboxylic acid or an anhydride of carboxylic acid, alone or in mixture with (H) a copolymer of polyolefin,
and wherein:
- the thickness of the contact layer is from 150 µm and 300 µm, preferably from 200 and 250 µm, more preferably from 225 µm and 245 µm,
- the thickness of the core layer is from 5 µm and 50 µm, preferably from 15 and 40 µm, more preferably from 20 µm and 30 µm, and
- the thickness of the outer layer is from 50 µm and 150 µm, in particular from 55 µm to 150 µm, preferably from 80 and 120 µm, more preferably from 90 µm and 110 µm.

2. The multilayer film according to claim 1, wherein the copolymer of ethylene and α-olefin (A) is a plastomer polyolefin having from 4 to 8 carbon atoms, in particular a linear low density copolymer of ethylene and oct-1-ene made from a process utilizing a metallocene catalyst.

3. The multilayer film according to claim 1 or 2, wherein polyolefin (B) is a homopolymer of ethylene, in particular a low density polymer of ethylene.

4. The multilayer film according to any one of claims 1 to 3, wherein the ethylene vinyl alcohol copolymer (C) is flex-crack resistant.

5. The multilayer film according to any one of claims 1 to 4, wherein the copolymer (D) is an acrylic acid copolymer of ethylene ionomer wherein the carboxylate groups are associated with zinc cations.

6. The multilayer film according to any one of claims 1 to 5, wherein the polyolefin (E) is a copolymer of ethylene with one or more olefin having from 4 to 8 carbon atoms, in particular a linear low density copolymer of ethylene and hexene.

7. The multilayer film according to any one of claims 1 to 6, wherein the copolymer (F) is a plastomer polyolefin having from 4 to 8 carbon atoms, in particular a linear low density copolymer of ethylene and oct-1-ene made from a process utilizing a metallocene catalyst.

8. The multilayer film according to any one of claims 1 to 7, wherein the copolymer (G) is a copolymer of ethylene grafted with maleic anhydride.

9. The multilayer film according to any one of claims 1 to 8, wherein the copolymer (H) is a plastomer polyolefin having from 4 to 8 carbon atoms, in particular a linear low density copolymer of ethylene and oct-1-ene made from a process utilizing a metallocene catalyst.

10. The multilayer film according to any one of claims 1 to 9, wherein the contact layer comprises a mixture of (A) and (B) with a mass ratio (B)/(A) less than or equal to 95/5, preferably in the range of 5/95 to 95/5, more preferably in the range of 0.2 to 5, even more preferably in the range of 0.2 to 2.

11. The multilayer film according to any one of claims 1 to 10, wherein the core layer comprises a mixture of (C) an ethylene vinyl alcohol copolymer having a content of ethylene in the range of 25 to 48 mol % and (D) an ionomer acid ethylene copolymer with a mass ratio (C)/(D) in the range of 95/5 to 55/45, preferably in the range of 10 to 19, more preferably in the range of 15 to 19.

12. The multilayer film according to any one of claims 1 to 11, wherein the outer layer comprises a mixture of (E) and (F) with a mass ratio (E)/(F) more than or equal to 5/95, preferably in the range of 5/95 to 100/0, more preferably in the range of 1 to 5, even more preferably in the range of 1 to 2.5.

13. The multilayer film according to any one of claims 1 to 12, wherein the first tie layer and second tie layer comprise independently of each other a mixture of (G) and (H) with a mass ratio (H)/(G) less than or equal to 95/5, preferably in the range of 5/95 to 95/5, more preferably in the range of 0.2 to 5, even more preferably in the range of 0.2 to 2.5.

14. The multilayer film according to any one of claims 1 to 13, wherein the contact layer contains no additives or limited amounts, preferably less than 0.10 wt %, more preferably less than 0.07 wt %, of additives able to release a degradation compound further to gamma irradiation that can slow down or delay cellular growth, such as Tris(2,4-ditert-butylphenyl) phosphite.

15. The multilayer film according to any one of claims 1 to 13, said multilayer film further comprising one or several intermediate layers between two of any layers of the film.

16. A process for manufacturing the multilayer film according to anyone of claims 1 to 15, comprising coextrusion of the contact layer, first tie layer, core layer, second tie layer and outer layer.

17. A single-use pouch whose wall comprises the multilayer film according to anyone of claims 1 to 15.

18. A bioreactor comprising the single-use pouch according to claim 17.

## Patentansprüche

1. Mehrschichtfolie zur Herstellung eines Beutels, wobei die Mehrschichtfolie umfasst:
- eine Kontaktschicht,
- eine Kernschicht,
- eine äußere Schicht,
wobei:
- die Kontaktschicht und die Kernschicht integral mit einer ersten Verbindungsschicht sind,
- die äußere Schicht und die Kernschicht integral mit einer zweiten Verbindungsschicht sind,
- die Kontaktschicht (A) ein Copolymer von Ethylen und α-Olefin mit einer Dichte im Bereich von 0,870 g/cm³ bis 0,910 g/cm³, allein oder im Gemisch mit (B) einem Polyolefin mit einer Dichte im Bereich von 0,910 g/cm³ bis 0,940 g/cm³ umfasst,
- die Kernschicht (C') ein biegerissbeständiges Ethylenvinylalkohol-Copolymer mit einem Gehalt von Ethylen im Bereich von 25 bis 48 Mol-% oder eine Mischung aus (C) einem Ethylenvinylalkohol-Copolymer mit einem Ethylengehalt im Bereich von 25 bis 48 Mol-% und (D) einem lonomer-Säure-Ethylen-Copolymer umfasst, wobei das Ethylenvinylalkohol-Copolymer biegerissbeständig ist, wenn die Anzahl der nach 100 Zyklen gemäß ASTM F392 (2004) gemessenen Poren kleiner oder gleich 30 ist, wobei der Test an einer 30 µm dicken Monoschicht bei 23 °C mit einem Gelbo-Flex-Prüfgerät und bei einer Drehbewegung von 440° durchgeführt wird,
- die äußere Schicht (E) ein Polyolefin mit einer Dichte im Bereich von 0,910 g/cm³ bis 0,940 g/cm³, allein oder in Mischung mit (F) einem Copolymer von Ethylen und α-Olefin mit einer Dichte im Bereich von 0,870 g/cm³ bis 0,910 g/cm³ umfasst
- die erste Verbindungsschicht und die zweite Verbindungsschicht unabhängig voneinander (G) ein Copolymer von Polyolefin, das mit einer Carbonsäure oder einem Carbonsäureanhydrid gepfropft ist, allein oder in Mischung mit (H) einem Copolymer von Polyolefin umfassen
und wobei:
- die Dicke der Kontaktschicht 150 µm bis 300 µm, vorzugsweise 200 µm bis 250 µm, besonders bevorzugt 225 µm bis 245 µm, beträgt,
- die Dicke der Kernschicht 5 µm bis 50 µm, vorzugsweise 15 µm bis 40 µm, besonders bevorzugt 20 µm bis 30 µm, beträgt, und
- die Dicke der äußere Schicht 50 µm bis 150 µm, insbesondere 55 µm bis 150 µm, vorzugsweise 80 µm bis 120 µm, besonders bevorzugt 90 µm bis 110 µm, beträgt.

2. Mehrschichtfolie nach Anspruch 1, wobei das Copolymer von Ethylen und α-Olefin (A) ein Plastomerpolyolefin mit 4 bis 8 Kohlenstoffatomen ist, insbesondere ein lineares Copolymer mit niedriger Dichte von Ethylen und Oct-1-en, hergestellt mit einem Verfahren unter Verwendung eines Metallocenkatalysators.

3. Mehrschichtfolie nach Anspruch 1 oder 2, wobei das Polyolefin (B) ein Homopolymer von Ethylen ist, insbesondere ein Ethylen-Polymer niedriger Dichte.

4. Mehrschichtfolie nach einem der Ansprüche 1 bis 3, wobei das Ethylenvinylalkohol-Copolymer (C) biegerissbeständig ist.

5. Mehrschichtfolie nach einem der Ansprüche 1 bis 4, wobei das Copolymer (D) ein Acrylsäurecopolymer von Ethylen-Ionomer ist, wobei die Carboxylatgruppen mit Zink-Kationen assoziiert sind.

6. Mehrschichtfolie nach einem der Ansprüche 1 bis 5, wobei das Polyolefin (E) ein Copolymer von Ethylen mit einem oder mehreren Olefinen mit 4 bis 8 Kohlenstoffatomen ist, insbesondere ein lineares Copolymer niedriger Dichte von Ethylen und Hexen.

7. Mehrschichtfolie nach einem der Ansprüche 1 bis 6, wobei das Copolymer (F) ein Plastomerpolyolefin mit 4 bis 8 Kohlenstoffatomen ist, insbesondere ein lineares Copolymer niedriger Dichte aus Ethylen und Oct-1-en, hergestellt mit einem Verfahren unter Verwendung eines Metallocenkatalysators.

8. Mehrschichtfolie nach einem der Ansprüche 1 bis 7, wobei das Copolymer (G) ein Copolymer von mit Maleinsäureanhydrid gepfropftem Ethylen ist.

9. Mehrschichtfolie nach einem der Ansprüche 1 bis 8, wobei das Copolymer (H) ein Plastomerpolyolefin mit 4 bis 8 Kohlenstoffatomen ist, insbesondere ein lineares Copolymer niedriger Dichte von Ethylen und Oct-1-en, hergestellt mit einem Verfahren unter Verwendung eines Metallocenkatalysators.

10. Mehrschichtfolie nach einem der Ansprüche 1 bis 9, wobei die Kontaktschicht eine Mischung aus (A) und (B) mit einem Massenverhältnis (B)/(A) von kleiner oder gleich 95/5, bevorzugt in dem Bereich von 5/95 bis 95/5, stärker bevorzugt in dem Bereich von 0,2 bis 5, noch stärker bevorzugt in dem Bereich von 0,2 bis 2 umfasst.

11. Mehrschichtfolie nach einem der Ansprüche 1 bis 10, wobei die Kernschicht eine Mischung aus (C) einem Ethylenvinylalkohol-Copolymer mit einem Ethylengehalt im Bereich von 25 bis 48 Mol-% und (D) einem Ionomer-Säure-Ethylencopolymer mit einem Massenverhältnis (C)/(D) in dem Bereich von 95/5 bis 55/45, bevorzugt in dem Bereich von 10 bis 19, stärker bevorzugt in dem Bereich von 15 bis 19, umfasst.

12. Mehrschichtfolie nach einem der Ansprüche 1 bis 11, wobei die äußere Schicht eine Mischung aus (E) und (F) mit einem Massenverhältnis (E)/(F) von mehr als oder gleich 5/95, bevorzugt in dem Bereich von 5/95 bis 100/0, stärker bevorzugt in dem Bereich von 1 bis 5, noch stärker bevorzugt in dem Bereich von 1 bis 2,5, umfasst.

13. Mehrschichtfolie nach einem der Ansprüche 1 bis 12, wobei die erste Verbindungsschicht und die zweite Verbindungsschicht unabhängig voneinander eine Mischung von (G) und (H) mit einem Massenverhältnis (H)/(G) von weniger als oder gleich 95/5, bevorzugt in dem Bereich von 5/95 bis 95/5, stärker bevorzugt in dem Bereich von 0,2 bis 5, noch stärker bevorzugt in dem Bereich von 0,2 bis 2,5, umfasst.

14. Mehrschichtfolie nach einem der Ansprüche 1 bis 13, wobei die Kontaktschicht keine Additive oder begrenzte Mengen, bevorzugt weniger als 0,10 Gew.-%, stärker bevorzugt weniger als 0,07 Gew.-% Additive enthält, die in der Lage sind, eine Abbauverbindung weiter zu gamma-Bestrahlung freizusetzen, die das Zellwachstum verlangsamen oder verzögern kann, wie Tris(2,4-ditert-butylphenyl)phosphit.

15. Mehrschichtfolie nach einem der Ansprüche 1 bis 13, wobei die Mehrschichtfolie ferner eine oder mehrere Zwischenschichten zwischen zwei beliebigen Schichten der Folie umfasst.

16. Verfahren zur Herstellung der Mehrschichtfolie nach einem der Ansprüche 1 bis 15, umfassend Coextrusion der Kontaktschicht, der ersten Verbindungsschicht, der Kernschicht, der zweiten Verbindungsschicht und der Außenschicht.

17. Einwegbeutel, dessen Wand die Mehrschichtfolie nach einem der Ansprüche 1 bis 15 umfasst.

18. Bioreaktor, umfassend den Einwegbeutel nach Anspruch 17.

## Revendications

1. Film multicouche pour la fabrication d'un sachet, ledit film multicouche comprenant :
- une couche de contact,
- une couche centrale,
- une couche extérieure,
dans lequel :
- la couche de contact et la couche centrale sont fabriquées d'un seul tenant avec une première couche de liaison,
- la couche extérieure et la couche centrale sont fabriquées d'un seul tenant avec une seconde couche de liaison,
- la couche de contact comprend (A) un copolymère d'éthylène et d'α-oléfine ayant une masse volumique dans la plage de 0,870 g/cm³ à 0,910 g/cm³, seul ou en mélange avec (B) une polyoléfine ayant une masse volumique dans la plage de 0,910 g/cm³ à 0,940 g/cm³,
- la couche centrale comprend (C') un copolymère d'éthylène et d'alcool vinylique résistant aux cassures par flexion ayant une teneur en éthylène dans la plage de 25 à 48 % en moles ou un mélange de (C) un copolymère d'éthylène et d'alcool vinylique ayant une teneur en éthylène dans la plage de 25 à 48 % en moles et de (D) un copolymère d'acide ionomère et d'éthylène, le copolymère d'éthylène et d'alcool vinylique étant considéré comme résistant aux cassures par flexion quand le nombre de perforations mesuré après 100 cycles selon la méthode ASTM F392 (2004) est inférieur ou égal à 30, le test étant effectué sur un échantillon de film monocouche de 30 µm, à 23 °C, avec un testeur Gelbo-flex et pour mouvement de torsion de 440°,
- la couche extérieure comprend (E) une polyoléfine ayant une masse volumique dans la plage de 0,910 g/cm³ à 0,940 g/cm³, seule ou en mélange avec (F) un copolymère d'éthylène et d'α-oléfine ayant une masse volumique dans la plage de 0,870 g/cm³ à 0,910 g/cm³,
- la première couche de liaison et la seconde couche de liaison comprennent indépendamment l'une de l'autre (G) un copolymère de polyoléfine greffé avec un acide carboxylique ou un anhydride d'acide carboxylique, seul ou en mélange avec (H) un copolymère de polyoléfine
et dans lequel :
- l'épaisseur de la couche de contact est comprise entre 150 µm et 300 µm, de préférence entre 200 µm et 250 µm, et plus préférentiellement entre 225 µm et 245 µm ;
- l'épaisseur de la couche centrale est comprise entre 5 µm et 50 µm, de préférence entre 15 µm et 40 µm, et plus préférentiellement entre 20 µm et 30 µm ; et
- l'épaisseur de la couche extérieure est comprise entre 50 µm et 150 µm, en particulier entre 55 µm et 150 µm, de préférence entre 80 µm et 120 µm, et plus préférentiellement entre 90 µm et 110 µm.

2. Film multicouche selon la revendication 1, dans lequel le copolymère d'éthylène et d'α-oléfine (A) est une polyoléfine plastomère ayant de 4 à 8 atomes de carbone, en particulier un copolymère linéaire à faible masse volumique d'éthylène et d'oct-1-ène fabriqué à partir d'un procédé utilisant un catalyseur métallocène.

3. Film multicouche selon les revendications 1 ou 2, dans lequel la polyoléfine (B) est un homopolymère d'éthylène, en particulier un polymère d'éthylène à faible masse volumique.

4. Film multicouche selon l'une quelconque des revendications 1 à 3, dans lequel le copolymère d'éthylène et d'alcool vinylique (C) résiste aux cassures par flexion.

5. Film multicouche selon l'une quelconque des revendications 1 à 4, dans lequel le copolymère (D) est un copolymère d'acide acrylique d'ionomère d'éthylène dans lequel les groupes carboxylates sont associés à des cations zinc.

6. Film multicouche selon l'une quelconque des revendications 1 à 5, dans lequel la polyoléfine (E) est un copolymère d'éthylène avec une ou plusieurs oléfines ayant de de 4 à 8 atomes de carbone, en particulier un copolymère linéaire à faible masse volumique d'éthylène et d'hexène.

7. Film multicouche selon l'une quelconque des revendications 1 à 6, dans lequel le copolymère (F) est une polyoléfine plastomère ayant de 4 à 8 atomes de carbone, en particulier un copolymère linéaire à faible masse volumique d'éthylène et d'oct-1-ène fabriqué à partir d'un procédé utilisant un catalyseur métallocène.

8. Film multicouche selon l'une quelconque des revendications 1 à 7, dans lequel le copolymère (G) est un copolymère d'éthylène greffé avec un anhydride maléique.

9. Film multicouche selon l'une quelconque des revendications 1 à 8, dans lequel le copolymère (H) est une polyoléfine plastomère ayant de 4 à 8 atomes de carbone, en particulier un copolymère linéaire à faible masse volumique d'éthylène et d'oct-1-ène fabriqué à partir d'un procédé utilisant un catalyseur métallocène.

10. Film multicouche selon l'une quelconque des revendications 1 à 9, dans lequel la couche de contact comprend un mélange de (A) et de (B) avec un rapport massique (B)/(A) inférieur ou égal à 95/5, de préférence dans la plage de 5/95 à 95/5, de manière davantage préférée dans la plage de 0,2 à 5, de manière encore davantage préférée dans la plage de 0,2 à 2.

11. Film multicouche selon l'une quelconque des revendications 1 à 10, dans lequel la couche centrale comprend un mélange de (C) un copolymère d'éthylène et d'alcool vinylique ayant une teneur en éthylène dans la plage de 25 à 48 % en moles et de (D) un copolymère d'acide ionomère et d'éthylène avec un rapport massique (C)/(D) dans la plage de 95/5 à 55/45, de préférence dans la plage de 10 à 19, de manière davantage préférée dans la plage de 15 à 19.

12. Film multicouche selon l'une quelconque des revendications 1 à 11, dans lequel la couche extérieure comprend un mélange de (E) et de (F) avec un rapport massique (E)/(F) supérieur ou égal à 5/95, de préférence dans la plage de 5/95 à 100/0, de manière davantage préférée dans la plage de 1 à 5, de manière encore davantage préférée dans la plage de 1 à 2,5.

13. Film multicouche selon l'une quelconque des revendications 1 à 12, dans lequel la première couche de liaison et la seconde couche de liaison comprennent indépendamment l'une de l'autre un mélange de (G) et de (H) avec un rapport massique (H)/(G) inférieur ou égal à 95/5, de préférence dans la plage de 5/95 à 95/5, de manière davantage préférée dans la plage de 0,2 à 5, de manière encore davantage préférée dans la plage de 0,2 à 2,5.

14. Film multicouche selon l'une quelconque des revendications 1 à 13, dans lequel la couche de contact ne contient pas d'additifs ou contient des quantités limitées, de préférence inférieures à 0,10 % en poids, de manière davantage préférée inférieures à 0,07 % en poids, d'additifs aptes à libérer un composé de dégradation à la suite d'une irradiation gamma qui peut ralentir ou retarder la croissance cellulaire, tels que le phosphite de tris (2,4-di-tert-butylphényle).

15. Film multicouche selon l'une quelconque des revendications 1 à 13, ledit film multicouche comprenant en outre une ou plusieurs couches intermédiaires entre deux couches quelconques du film.

16. Procédé de fabrication du film multicouche selon l'une quelconque des revendications 1 à 15, comprenant la coextrusion de la couche de contact, de la première couche de liaison, de la couche centrale, de la seconde couche de liaison et de la couche extérieure.

17. Sachet à usage unique dont la paroi comprend le film multicouche selon l'une quelconque des revendications 1 à 15.

18. Bioréacteur comprenant le sachet à usage unique selon la revendication 17.
